# EUROPÄISCHE PATENTSCHRIFT

(11) **EP 0 303 946 B1**
(45) Veröffentlichungstag und Bekanntmachung des Hinweises auf die Patenterteilung: **31.03.1993**
(21) Anmeldenummer: 88112936.5
(22) Anmeldetag: 09.08.1988
(51) Int. Cl.: A23C 9/123, A23C 11/10, A23L 1/03, A23K 1/00

(54) **Rekonstituierbares festes Trockenprodukt zum Direkt-Verzehr, Verfahren zu seiner Herstellung und seine Verwendung**
Reconstitutable solid dry product for direct consumption, process for its preparation and its use
Produit sec solide à reconstituer et à consommation directe, son procédé de préparation et son utilisation

(30) Priorität: 21.08.1987 DE 3727946
(43) Veröffentlichungstag der Anmeldung: 22.02.1989
(73) Patentinhaber: Munk, Werner Georg, D-88267 Vogt (DE)
(72) Erfinder: Munk, Werner Georg, D-88267 Vogt (DE)
(74) Vertreter: Kraus, Walter, Dr.

(56) Entgegenhaltungen:
- DE-A- 2 024 018
- DE-A- 3 132 563
- FR-A- 2 479 689
- FR-A- 2 560 046
- FR-A- 2 598 889
- GB-A- 2 043 101
- J.Lj. RASIC et al.: "Bifidobacteria and their role", 1983, Seiten 138-143, Birkhäuser Verlag, Basel, CH
- Lebensmittellexikon VEB S. 32

## Beschreibung

Die Erfindung betrifft ein durch den Speichel rekonstituierbares, bei Raumtemperatur lagerfähiges Trockenprodukt in fester Form mit einem Gehalt an Lactulose unter 0,5 Gew.-%, bezogen auf das Trockenprodukt, zum Direktverzehr auf der Basis von Milch oder Milchprodukten und mit einem Gehalt an Milchsäurebakterien, ein Verfahren zu seiner Herstellung und seine Verwendung.

Die Milchsäurebakterien, d.h. anaerobe Bakterien, die beim Abbau von Kohlehydraten als Hauptendprodukt Milchsäure (Lactat) ausscheiden, sind ein wesentlicher Bestandteil der Darmflora. Insbesondere ist es wichtig, daß die Population der Bifidobakterien, die zu den Milchsäurebakterien gehören, im Darm ausreicht. Die Bifidobakterien werden dem Dickdarm des Säuglings mit der Muttermilch zugeführt, und bereits beim Säugling bildet sich eine kräftige und infektionshemmende Bifidoflora im Darm aus. Es ist bekannt, daß die Darmflora durch zahlreiche äußere Einflüsse gestört und geschädigt werden kann. Störungen der Darmflora können beispielsweise durch Antibiotika, Sulfonamide, Antidiabetika und durch Bestrahlung entstehen. Auch bei fermentativ und bakteriell bedingten Verdauungsstörungen, intestinalen Intoxikationen und deren Folgen wird die Darmflora gestört. Es besteht daher ein Bedarf nach Mitteln, mit denen die gestörte bzw. geschädigte Darmflora wieder intakt gebracht werden kann.

Auch bei gesunden Personen ist es günstig, wenn die Population an Milchsäurebakterien, insbesondere Bifidobakterien, im Darm aufgefrischt und erneuert wird. Werden Präparate, mit denen die Milchsäurebakterienkonzentration im Darm beeinflußt werden soll, oral verabreicht, ist es erforderlich, daß diese Präparate die Magenpassage überwinden. Die Milchsäurekulturen zersetzen sich im Magen, und damit eine bestimmte Milchsäurebakterienkonzentration im Darm erreicht wird, muß die Milchsäurebakterien-, insbesondere die Bifidobakterienkonzentration, möglichst hoch sein.

Milchsäurebakterien kommen in Nahrungsmitteln oder Getränken vor, die aus Pflanzen gewonnen werden, wie z.B. in Sauerkraut, Wein und Bier und in Milch und Milchprodukten, wie beispielsweise in Butter, Käse, Buttermilch und Joghurt. Von diesen Produkten ist der Verbrauch an Milch und Milchprodukten, insbesondere an Buttermilch und Joghurt, in den letzten Jahren stark angestiegen, da das Gesundheitsbewußtsein in der Bevölkerung zugenommen hat. Der Verbrauch an Joghurt hat sich in den letzten Jahren vervielfacht.

Die Konservierung milchsäurebildender Bakterien erfolgt üblicherweise durch Kühlung oder Tiefkühlung oder im trockenen Zustand als zerstäubungs- oder gefriergetrocknetes Produkt, welches Nahrungs- oder Arzneimitteln beigemischt werden kann, die nicht auf die z.B. für Bifidobakterien letale Temperatur erhitzt werden, d.h. die Zumischung erfolgt kurzfristig vor dem Verzehr.

Es sind verschiedene Produkte bekannt, welche Milchsäurebakterien enthalten und zur Behandlung von Störungen der Darmflora bei Antibiotika , Sulfonamid-, Antidiabetika und Strahlenschäden, fermentativ und bakteriell bedingten Verdauungsstörungen und deren Folgen verwendet werden. Die im Handel erhältlichen Produkte besitzen jedoch verschiedene Nachteile. Einige der bekannten Produkte sind bei Raumtemperatur nicht lagerfähig. Der wesentlichste Nachteil der bekannten Produkte ist jedoch der, daß die Konzentration an Milchsäurebakterien zu niedrig ist. Die Milchsäurebakterien passieren den Magen nur teilweise, und die Konzentration der Milchsäurebakterien, die im Darm erreicht wird, ist nicht ausreichend hoch. Es ist ein weiterer Nachteil, daß ein Teil der bekannten Produkte Zusatzstoffe zur Konservierung enthält. Die Konservierung milchsäurebildender Bakterien erfolgt üblicherweise durch Kühlung oder Tiefkühlung.

In der EP-A-9 219 werden trockene chipsartige Nahrungs-oder Genußmittelscheiben und Verfahren zu ihrer Herstellung beschrieben Diese bekannten Produkte sind aufgrund ihrer Form und Konsistenz zum unmittelbaren Mundverzehr geeignet. Sie werden hergestellt, indem eine wäßrige Grundmasse aus Lebensmittelrohstoffen, die in Wasser lösliche wie auch unlösliche Gerüstbildner enthält, gegebenenfalls unter Zugabe von Geschmacks- und Würzstoffen, in eine verzehrbereite Zusammensetzung verarbeitet wird, die eine noch fließfähige dickflüssige bis plastisch verformbare Konsistenz aufweist. Die Masse wird zu Scheiben mit einer Dicke von 1 bis 10 mm geformt und dann unter Ausbildung eines porösen Trockenproduktes schonend getrocknet. Als Trocknung wird eine Vakuumtrocknung oder Gefriertrocknung bei einer Temperatur bis maximal 65°C durchgeführt.

Die bekannte Masse kann aus einer Grundmasse aus Milchprodukten hergestellt werden und kann Joghurtkulturen enthalten. Das aus einer solchen Grundmasse hergestellte bekannte Produkt in Chipsform besitzt den Nachteil, daß die Konzentration an Milchsäurebakterien bei ca. 10⁵/g liegt. Diese Konzentration ist nicht zufriedenstellend.

In der DE-OS 31 32 563 wird ein Verfahren zur Herstellung von gefriergetrockneten Produkten, insbesondere Lebensmitteln, beschrieben, bei dem die Produkte vor der Gefriertrocknung in eine gefriertrockenfähige Form gebracht und durch Kühlung mit flüssigem Stickstoff vorgefrostet werden. Der gasförmige Stickstoff kann gleichzeitig als Spülgas bei der Portionierung und bei der Gefriertrocknung verwendet werden. Wird dieses bekannte Verfahren zur Herstellung von gefriergetrockneten Produkten aus Milch oder Milchprodukten, wie zum Beispiel Joghurt, verwendet, so werden Produkte erhalten, deren Konzentration an Milchsäurebakterien nicht ausreichend hoch ist.

In der DE-PS 27 55 037 C2 wird ein pulverförmiges Mittel beschrieben, welches 28 bis 57 Gew.-% Lactulose und mindestens 8 x 10¹⁰ gefriergetrocknete Zellen von Mikroorganismen der Gattung Bifidobakterien enthält. Gemäß dieser Druckschrift wird die Lebensfähigkeit der Bifidobakterien durch den Gehalt an Lactulose erhöht. Dieses Produkt besitzt den Nachteil, daß Lactulose praktisch als Inertstoff dem Produkt zugemischt werden soll und daß es nur als pulverförmiges Mittel vorliegt. Das pulverförmige Mittel muß zum Gebrauch in Wasser gelöst bzw. suspendiert werden. Dieses bekannte Produkt besitzt den Nachteil, daß es nicht zum Direktverzehr geeignet ist, sondern erst in Wasser gelöst bzw. suspendiert werden muß. Es besitzt weiterhin den Nachteil, daß es erforderlich ist, das pulverförmige Produkt an einem kühlen und dunklen Ort aufzubewahren, da das Gemisch eine hohe Hygroskopizität aufweist. Das pulverförmige Produkt muß daher luftdicht verpackt oder in Kapseln gefüllt werden und danach luftdicht verpackt werden, bevor es vertrieben wird.

Gegenstand der FR-A-2 479 689 ist eine Tablette, welche Bifidobakterien enthält und dadurch gekennzeichnet ist, daß sie weiterhin mehrere Substanzen aus der Gruppe Stärke, Stärkehydrolysat und Proteine und weniger als 4% Wasser enthält. Die längerdauernde Lebensfähigkeit der Bifidobakterien wird hier auf die Auswahl einer oder mehrerer Substanzen aus der Gruppe Stärke, Stärkehydrolysat und Proteine zurückgeführt.

Gegenstand der FR-A-2 560 046 ist ein diätetisches Produkt zur Verminderung der Harnstoffkonzentration in physiologischen Flüssigkeiten, das dadurch gekennzeichnet ist, daß es Urease enthaltende Bifidobakterien aus der Gattung Bifidobacterium suis oder infantis enthält.

Die GB-A-2 043 101 betrifft ein Verfahren zur Herstellung von Lebensmitteln und Getränken, welche Bifidobakterien enthalten, das dadurch gekennzeichnet ist, daß man Bifidobakterien oder Bifidobakterien und Milchsäurebakterien in ein Medium inokuliert und kultiviert, welches ein Gemisch, welches Reis, der zur α-Stärketransformation behandelt ist, und Zucker, die durch Bifidobakterien fermentierbar sind, enthält, oder weitere Milchbestandteile zusätzlich zu den obigen Inhaltsstoffen enthält, umfaßt, und gegebenenfalls das kultivierte Produkt weiterverarbeitet.

Gegenstand der DE-OS 20 24 018 ist ein Verfahren zur Herstellung von trockenem bulgarischen Joghurt in Form von Tabletten oder Pulver, das dadurch gekennzeichnet ist, daß die Kuhmilch in Ansäuerung, die einen an Antibiotika angepaßten Stamm von Lactobacterium bulgaricum 2T Nr. 236 und an Lyophilisationsverhältnisse angepaßten Stamm von Lactobacterium bulgaricum F1 Nr. 237 und einen Stamm von Streptococcus thermophylus 26T Nr. 240 enthält, angesäuert wird, anschließend mit Lactobacterium bulgaricum angereichert, wonach die fermentierte, angesäuerte Milch eingefroren, getrocknet und in an sich bekannter Weise verpackt wird.

Der vorliegenden Erfindung liegt die Aufgabe zugrunde, ein Trockenprodukt zum Direktverzehr in fester Form, d.h. nicht in pulverförmiger Form, auf der Basis von Milch oder Milchprodukten zur Verfügung zu stellen, welches einen hohen Gehalt an Milchsäurebakterien, insbesondere Bifidobakterien, aufweist. Die Milchsäurebakterien sollen reanimations- als auch populationsfähig sein. Das Produkt soll durch den Speichel rekonstituierbar sein. Es soll bei Raumtemperatur mindestens drei Monate lagerfähig sein, ohne daß sich der Gehalt an Milchsäurebakterien wesentlich ändert. Das Produkt soll keine zugesetzte Lactulose enthalten. Der Gehalt an Lactulose soll, bezogen auf das Produkt, unter 0,5 Gew.-% liegen.

Erfindungsgemäß soll ein Produkt zur Verfügung gestellt werden, das auch als Kultur bzw. als Halbfertigerzeugnis zur Joghurtherstellung verwendet werden kann. Das erfindungsgemäße Produkt soll als Nahrungsmittel, Arzneimittel, Dätetikum oder als Zusatz zu Nahrungsmitteln verwendet werden. Es soll weiterhin als Futter oder Futterzusatzstoff für Tiere verwendet werden.

Weiterhin soll ein Verfahren zu seiner Herstellung zur Verfügung gestellt werden, welches auf einfache Weise durchzuführen ist.

Gegenstand der Erfindung ist ein Produkt der eingangs genannten Art, das dadurch gekennzeichnet ist, daß der Gehalt an Milchsäurebakterien mindestens 10⁸/g Substanz beträgt und daß das Produkt eine feinporöse Außenstruktur besitzt, wodurch die Milchsäurebakterien während einer längerdauernden Lagerzeit lebensfähig bleiben.

Es ist bevorzugt, daß der Gehalt an Milchsäurebakterien im Bereich von 10⁸/g bis 10¹²/g, besonders bevorzugt im Bereich von 10⁹/g bis 10¹⁰/g, liegt.

Ein besonders bevorzugtes erfindungsgemäßes Produkt besitzt einen Gehalt an Milchsäurebakterien nach dem Lagern während mindestens drei Monaten bei Raumtemperatur im Bereich von 10⁸/g bis 10¹⁰/g, besonders bevorzugt von mindestens 10⁹/g.

Das erfindungsgemäße Produkt liegt in Form von Scheiben, Chips, Stäbchen, Figuren und gegebenenfalls mit einem Überzug, wie beispielsweise aus Zucker, Schokolade etc., versehen,vor.

Die Erfindung betrifft weiterhin ein Verfahren zur Herstellung des Produktes unter Herstellung einer wäßrigen Grundmischung auf der Basis von Milch bzw. Milchprodukten, Kohlehydraten und gegebenenfalls weiteren Zusatzstoffen, Homogenisierung, Erhitzung, Kühlung, Beimpfung, Bebrütung und Blitzfrostung mit flüssigem Stickstoff, das dadurch gekennzeichnet ist, daß die Mischung unter Inertgasatmosphäre fließverformt wird, das verformte Produkt unter Inertgasatmosphäre blitz- bzw. schockgefrostet wird, das gefrorene Produkt unter Inertgasatmosphäre gesammelt wird, gegebenenfalls unter Inertgasatmosphäre zwischengelagert, unter Inertgasatmosphäre gefriergetrocknet und unter Inertgasatmosphäre gas- und aromadicht verpackt wird.

Es ist bevorzugt, daß bei allen Stufen als Inertgas Stickstoff verwendet wird, da man den Stickstoff der Schockfrostung bei den Verformungs-, Gefriertrocknungs- und Verpackungsstufen verwenden kann. Es ist jedoch möglich, auch andere Inertgase zu verwenden. Die Blitzfrostung wird so durchgeführt, daß das Produkt mindestens eine Kerntemperatur von -15°C, vorzugsweise eine Kerntemperatur im Bereich von -15°C bis -50°C, erreicht. Die Zwischenlagerung kann, muß aber nicht, durchgeführt werden. Sie wird dann durchgeführt, wenn die Gefriertrocknungsanlage noch besetzt ist. Die Zwischenlagerung wird bei einer Temperatur im Bereich von -15°C bis -50°C durchgeführt. Die Gefriertrocknung wird bei einem Druck im Bereich von 0,1 bis 50 mbar, vorzugsweise bei einem Druck im Bereich von 1 bis 5 mbar, und bei einer solchen Temperatur durchgeführt, daß eine Produkttemperatur von +45°C nicht überschritten wird. Es ist bei dem erfindungsgemäßen Verfahren wesentlich, daß die Produkttemperatur von +45°C nicht überschritten wird. Der Restwassergehalt sollte am Ende der Trocknung <5%, optimal 2 bis 3%, betragen.

Zur optimalen Lagerung erfolgt die Verpackung des getrockneten Produktes in vorzugsweise aluminiumbeschichteter Polypropylenfolie unter Stickstoffatmosphäre. Es ist wesentlich, daß das erfindungsgemäße Produkt gas- bzw aromadicht in einem lichtundurchlässigen Material verpackt wird.

Als Milch oder Milchprodukte kann man Milch aller Fettgehaltsstufen, Milchkonzentrate, wie eingedickte Mager-oder Vollmilch, mit Sojaprotein angereichterte Produkte, wie Sojamilch, und milchähnliche Nährmedien verwenden.

Beispiele für Kohlehydrate sind Zucker, insbesondere kristallisierbare, wie Glucose, Saccharose, Fructose, Lactose, Zuckeraustauschstoffe, wie Zuckeralkohole, Stärke und Stärkeprodukte einschließlich Stärkeabbauprodukte, Getreide bzw. Zerealien in zerkleinerter Form, Vegetabilien, wie Früchte, Nüsse und geeignete Gemüse sowie deren zubereitete Form. Das erfindungsgemäße Produkt enthält keine Lactulose oder Lactulose nur in einem Bereich von 40 bis 100 ppm. In anderen Worten, ist das erfindungsgemäße Produkt lactulosefrei oder lactulosearm.

Erfindungsgemäß enthält das Produkt Milchsäurebakterien. Bevorzugt enthält es Mikroorganismen der Gattung Bifidobakterien, die zu den Milchsäurebakterien gehören.

Als Zusatzstoffe können all die Zusatzstoffe verwendet werden, wie sie derzeit auf dem Nahrungsmittelgebiet eingesetzt werden. Beispiele sind Verdickungsmittel, Emulgatoren, Schaumstabilisatoren, Aroma-, Farb- und Geschmacksstoffe, Mineralstoffe und Spurenelemente.

Es war überraschend und hat nicht nahegelegen, daß man durch Blitzfrostung mit flüssigem Stickstoff und Arbeiten in einer Inertgasatmosphäre ein Produkt erhält, welches eine Milchsäurebakterienkonzentration nach dem Lagern während mindestens drei Monaten bei Raumtemperatur im Bereich von 10⁸/g bis 10¹⁰/g erhält. Die nach dem erfindungsgemäßen Verfahren hergestellten Produkte besitzen die Eigenschaft, während längerer Lagerdauer ihre ernährungsphysiologischen Eigenschaften nicht einzubüßen. Die im Produkt enthaltenen Milchsäurebakterien bleiben über Wochen bzw. Monate in hoher Konzentration lebens- und vermehrungsfähig, dienen einer natürlichen Anreicherung der Darmflora, aber auch zur Ergänzung beim Verzehr von thermisierten (haltbaren) Milchprodukten.

Es ist bevorzugt, nach dem erfindungsgemäßen Verfahren Produkte in Chipsform herzustellen, wobei auf die Offenbarung in der EP-A-9219 und in der DE-OS 31 32 563 expressis verbis Bezug genommen wird. Der Vorteil der Chips liegt darin, daß in einem Chip (≙ 1 g) mindestens derselbe Anteil reanimations- und populationsfähiger Bifidobakterien enthalten ist, wie in einem 100-g-Becher handelsüblichem Joghurt mit nachweisbarem Bifidobakterium.

Gleichzeitig wird bei einem 1-g-Chip durch das geringe Eigengewicht und des hohen Quellvermögens und einer hohen Volumenvergrößerung mit dem Mundspeichel ein annähernd gleiches Sättigungsgefühl wie beim Verzehr von 100 g frischem Joghurt erzielt. Während des Kauprozesses wird der Speichelfluß erhöht, angeregt durch den Schmelzvorgang der feinporigen Struktur des Trockenproduktes. Somit wird bei geringer Kalorienzufuhr ein Effekt erzielt, der bewirkt, daß sich das Produkt ideal als Schlankheitskost eignet.

Die Herstellung des Chips erfolgt im 1. Schritt genauso wie die eines Joghurts, jedoch mit der zur Gefriertrocknung notwendigen Anreicherung der Trockenmasse, d.h. Milch, Magermilchpulver und Zucker erhitzen, auf Bebrütungstemperatur abkühlen, mit bifidohaltiger Joghurtkultur beimpfen, bis zum gewünschten pH-Wert (vorzugsweise 4,9) bebrüten und nach Kühlung (+4°C) durch die in der Anlage beschriebene Fließformung für die Gefriertrocknung portionieren.

Die Gefriertrocknung hat so zu erfolgen, daß die Produktoberflächentemperatur nicht die maximale Bebrütungstemperatur des in der Trocknung befindlichen Bakteriums zu seiner Erhaltung übersteigt.

Die auf diese Weise hergestellten Joghurtchips mit einer Höhe von max. 10 mm, vorzugsweise 2 bis 5 mm, und einem Durchmesser von vorzugsweise 20 bis 30 mm haben ein Trockengewicht von ca. 1 g und enthalten mindestens 1 x 10¹⁰ nachweisbare Bifidobakterien pro g, die sich nach 3monatiger Lagerung bei Raumtemperatur auf 1 x 10⁹/g reduzierten. Die Reanimations- und Populationsfähigkeit der Joghurtbakterien kann dadurch nachgewiesen werden, daß die gefriergetrockneten Chips als "Kultur" zur Joghurtherstellung verwendet werden können.

Frischer Joghurt mit nachweisbaren Bifidobakterien enthält bei einer Lagerdauer von 3 Wochen bei +8°C bis +10°C im Mittel 1 x 10⁵ nachweisbare Bifidobakterien pro g, d.h. ein Bifidojoghurtchip enthält mindestens die 10.000fache Anzahl an Bifidobakterien in einem g oder die mindestens 100fache Menge als ein 100 g-Becher mit frischem Joghurt. Zur Aufnahme derselben Menge an Bifidobakterien wie von 5 Joghurtchips (à 1 g) pro Tag müßten somit mindestens 500 Becher (à 100 g) pro Tag an frischem Joghurt verzehrt werden.

Die nach dem erfindungsgemäßen Verfahren hergestellten Produkte besitzen die Eigenschaft, während längerer Lagerdauer ihre ernährungsphysiologischen Eigenschaften nicht einzubüßen. Die im Produkt enthaltenen Milchsäurebakterien bleiben über Wochen bzw. Monate in hoher Konzentration lebens- und vermehrungsfähig, dienen einer natürlichen Anreicherung der Darmflora, aber auch zur Ergänzung beim Verzehr von thermisierten (haltbaren) Milchprodukten Anhand der beigefügten Zeichnungen wird die Erfindung näher erläutert.

Es zeigen:

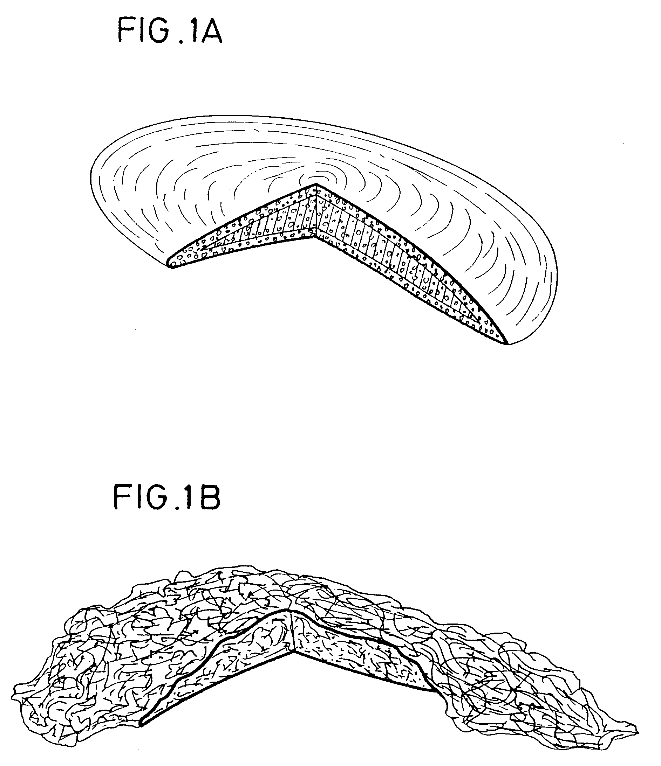

Figur 1A ein erfindungsgemäßes festes porenhaltiges Produkt und
Figur 1B das lose pulverförmige bekannte Produkt.

Man nimmt an, daß die warmlagerfähige Konservierung der Bakterien durch die feinporöse Struktur erzielt wird, da diese feine Porenstruktur als "Schutzwand" dient.

Die mit den Oberflächenporen eingelagerten Bakterien werden wie bei einem sprühgetrockneten Pulver durch Außen einflüsse während der Warmlagerung geschädigt, bilden damit aber einen wirksamen Abwehrschild für die im Porensystem eingelagerten Bakterien.

Durch diese Schutzwirkung bleiben die Bakterien im Inneren des Chips im Vergleich zu sprühgetrocknetem Pulver bei Raumtemperatur weitaus länger lebens- und vermehrungsfähig, d.h. die vorhandene Konzentration an Bakterien nimmt im Vergleich zum Pulver nach z.B. 3 Monaten nur um eine Zehnerpotenz, hier von 10¹⁰ auf 10⁹/g, ab, während die Konzentration in handelsüblichen pulverförmigen Produkten stärker dezimiert wird.

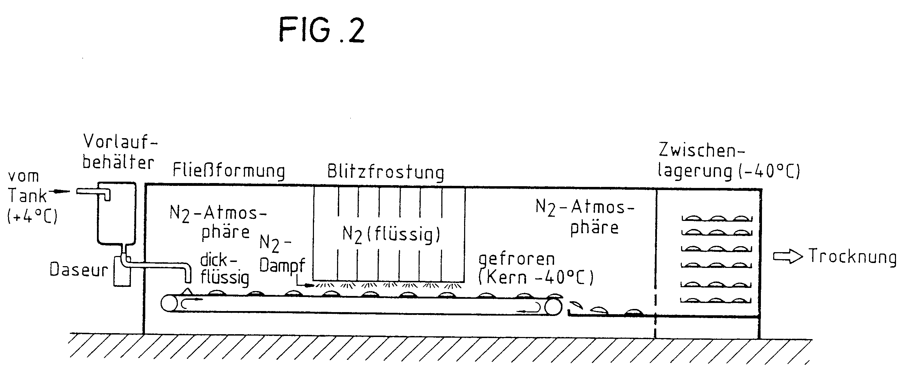

Fig. 2 zeigt eine schematische Darstellung der erfindungsgemäßen Fließverformung mit Blitzfrostung.

Von einem Tank wird die Grundmasse in einen Vorlaufbehälter geleitet. Über eine Dosiereinrichtung wird die Masse unter N₂-Atmosphäre auf ein Endlosband portioniert. Die einzelnen portionierten Teile werden mit flüssigem Stickstoff der Blitzfrostung unterworfen und unter N₂-Atmosphäre, gegebenenfalls nach Zwischenlagerung, der Gefriertrocknung zugeführt.

Die erfindungsgemäß hergestellten Produkte finden Verwendung als Zusatz zu Nahrungsmitteln, als Arzneimittel, Diätetikum, für Menschen und Tiere sowie als Futter- oder Futterzusatzstoff und als Halbfertigungserzeugnis zur Herstellung von Joghurt sowie als im Mund quellfähige Schlankheitskost.

Im folgenden wird die Herstellung von Naturjoghurtscheiben mit Traubenzucker allgemein erläutert.

### Naturjoghurtscheiben mit Traubenzucker

### Allgemeine Rezeptur:

| | |
|---|---|
| Vollmilch 3,5% Fett | 77% |
| Magermilchpulver | 9% |
| Traubenzucker | 8% |
| Speisestärke | 1% |
| Joghurt-Betriebskultur | 5% |

### Herstellung:

- Magermilchpulver, Traubenzucker und Stärke in die kalte Milch einrühren;
- Erhitzung auf +130°C/s (mit Vorhomogenisierung 200 bar bei +60°C);
- Kühlung auf +45°C;
- Beimpfung (Zugabe bifidohaltiger Kultur, Mischen);
- Bebrütung bei +43°C (bis 1,5% Milchsäure, ca. pH 4,9);
- Kühlung auf +4°C (unter Rühren);
- Formgebung nach Fließformung;
- Zwischenlagerung (falls erforderlich);
- Gefriertrocknung;
- Verpackung.

Die folgenden Beispiele erläutern die Erfindung.

### Beispiel 1

### Naturjoghurtscheiben mit Traubenzucker

Eine Trockenmischung, bestehend aus 11,7 kg Magermilchpulver, 10,4 kg Traubenzucker und 1,3 kg Speisestärke, wird unter langsamer Zugabe in 100 kg kalte Vollmilch (3,5% Fett) eingerührt. Der Ansatz wird auf +60°C vorgewärmt, bei dieser Temperatur mit 200 bar homogenisiert, auf +130°C kurzzeiterhitzt, auf +45°C gekühlt, 6,5 kg bifidohaltige Kultur in den Fließstrom zudosiert und in einen Stickstoffatmosphäre enthaltenden Bebrütungstank eingefahren, wobei die von dem einfließenden Ansatz verdrängte Stickstoffatmosphäre durch einen Sterilfilter entweichen kann und im Kopfraum des Bebrütungstankes ein geringer Stickstoffüberdruck zur anaeroben Bebrütung entsteht.

Nach Bebrütung bei +43°C bis 1,5% Milchsäuregehalt (≙ ca. pH 4,9) wird unter Rühren auf +4°C gekühlt, unter Stickstoffatmosphäre: fließverformt, blitzgefrostet, evtl. zwischengelagert und nach der Gefriertrocknung unter Stickstoff verpackt.

### Beispiel 2

### Naturjoghurtscheiben mit Fruchtzucker

1,5 kg Magermilchpulver (hemmstofffrei) werden mit 1 kg Fructose und 200 g Gelatine gut vermischt und in 6 kg Wasser unter langsamer Zugabe eingerührt.

Dieser Ansatz wird im Dampftopf auf +95°C erhitzt und 30 Minuten bei dieser Temperatur heißgehalten.

Nach Abkühlung auf +37°C (im Eiswasserbad unter Rühren) wird mit 1,5 kg bifidohaltiger Kultur beimpft und bei +37°C bis zum pH-Wert 4,6 bebrütet, danach im Eiswasserbad gekühlt auf <+10°C.

Nach der Beimpfung muß der Kopfraum des Bebrütungsgefäßes mit Inertgas zur Sauerstoffverdrängung bespült werden.

Nach erfolgter Kühlung wird die Joghurtmasse durch kräftiges Rühren glattgezogen und auf ±0°C gekühlt (Lufteinzug vermeiden).

Die gekühlte Masse wird mit einer sterilen Pipette auf ein vorgefrostetes Blech (mindestens -40°C) in 5-ml-Portionen aufgetragen, wobei sie sich scheibenförmig ausbreiten kann.

Nach anschließender Tieffrostung bei -55°C über 24 Stunden wird bei <2 mbar und maximal +35°C gefriergetrocknet.

### Beispiel 3

### Milchchips I

Magermilchkonzentrat mit einem Trockenmassegehalt von ca. 27% (≙ 3fach konzentriert) wird mit 20% bifidohaltiger Kultur beimpft und bei +36°C bis zu einem pH-Wert = 4,9 unter Stickstoffatmosphäre bebrütet.

Die gesäuerte Masse wird in Stickstoffatmosphäre gekühlt und glattgerührt, anschließend fließverformt, gefrostet und gefriergetrocknet.

### Beispiel 4

Man arbeitet wie in Beispiel 3 beschrieben und verwendet anstelle des Magermilchkonzentrats ein Vollmilchkonzentrat mit einem Trockenmassegehalt von 40%.

### Beispiel 5

### Milchchips II

10 kg kurzzeiterhitzte Milch wird bei +37°C mit 0,8 kg bifidohaltiger Joghurtkultur vermischt und bei +37°C bis pH 4,6 anaerob (unter Luftausschluß) bebrütet.

Die dickgelegte Masse wird im Vakuum-Rotationsverdampfer bei +35°C auf einen Trockenmassegehalt von 30 bis 40% durch Wasserentzug eingeengt, danach durch Fließformung, Frostung und Gefriertrocknung wie in Beispiel 1 zum Endprodukt verarbeitet.

## Patentansprüche

1. Durch den Speichel rekonstituierbares, bei Raumtemperatur lagerfähiges Trockenprodukt in fester Form mit einem Gehalt an Lactulose unter 0,5 Gew.-%, bezogen auf das Trockenprodukt, zum Direktverzehr auf der Basis von Milch oder Milchprodukten, Kohlehydraten, gegebenenfalls weiteren Zusatzstoffen und mit einem Gehalt an Milchsäurebakterien, dadurch **gekennzeichnet,** daß der Gehalt an Milchsäurebakterien mindestens 10⁸/g Substanz beträgt und daß das Produkt eine feinporöse Außenstruktur besitzt, wodurch die Milchsäurebakterien während einer längerdauernden Lagerzeit lebensfähig bleiben.

2. Trockenprodukt nach Anspruch 1, dadurch **gekennzeichnet,** daß es als Milch oder Milchprodukt Milch aller Fettgehaltsstufen, Milchkonzentrate, wie eingedickte Magermilch oder Vollmilch, mit Sojaprotein angereicherte Produkte, wie Sojamilch und milchähnliche Nährmedien, oder Gemische dieser Verbindungen enthält.

3. Trockenprodukt nach Anspruch 1 oder 2, dadurch **gekennzeichnet,** daß es als Kohlehydrate Zucker, Stärke, Stärkeprodukte einschließlich Stärkeabbauprodukte, Getreide bzw. Zerealien in zerkleinerter Form, Vegetabilien oder Gemische dieser Verbindungen enthält.

4. Trockenprodukt nach Anspruch 3, dadurch **gekennzeichnet,** daß es als Zucker Glucose, Saccharose, Fructose, Lactose, Zuckeraustauschstoffe oder Gemische dieser Verbindungen enthält.

5. Trockenprodukt nach mindestens einem der Ansprüche 1 bis 4, dadurch **gekennzeichnet,** daß der Gehalt an Milchsäurebakterien im Bereich von 10⁸/g bis 10¹²/g liegt.

6. Trockenprodukt nach mindestens einem der Ansprüche 1 bis 5, dadurch **gekennzeichnet,** daß der Gehalt an Milchsäurebakterien nach dem Lagern während mindestens 3 Monaten bei Raumtemperatur im Bereich von 10⁸/g bis 10¹⁰/g liegt.

7. Trockenprodukt nach mindestens einem der Ansprüche 1 bis 6, dadurch **gekennzeichnet,** daß es in Form von Scheiben, Chips, Stäbchen, Figuren, gegebenenfalls mit einem Überzug versehen, vorliegt.

8. Verfahren zur Herstellung des Produktes nach einem der Ansprüche 1 bis 7 unter Herstellung einer wäßrigen Grundmischung auf der Basis von Milch bzw. Milchprodukten, Kohlehydraten und gegebenenfalls weiteren Zusatzstoffen, Homogenisierung, Erhitzung, Kühlung, Beimpfung, Bebrütung und Blitzfrostung mit flüssigem Stickstoff, dadurch **gekennzeichnet,** daß die Mischung, unter Inertgasatmosphäre bebrütet, fließverformt wird, das verformte Produkt unter Inertgasatmosphäre blitz- bzw. schockgefrostet wird, das gefrorene Produkt unter Inertgasatmosphäre gesammelt wird, gegebenenfalls unter Inertgasatmosphäre zwischengelagert, unter Inertgasatmosphäre gefriergetrocknet und unter Inertgasatmosphäre gas- und aromadicht verpackt wird.

9. Verfahren nach Anspruch 8, dadurch **gekennzeichnet,** daß bei allen Stufen als Inertgas Stickstoff verwendet wird.

10. Verfahren nach einem der Ansprüche 8 oder 9, dadurch **gekennzeichnet,** daß die Blitzfrostung so durchgeführt wird, daß das Produkt eine Kerntemperatur von mindestens -15°C erreicht.

11. Verfahren nach einem der Ansprüche 8 bis 10, dadurch **gekennzeichnet,** daß die Blitzfrostung so durchgeführt wird, daß das Produkt eine Kerntemperatur im Bereich von -15 bis -50°C erreicht.

12. Verfahren nach einem der Ansprüche 8 bis 11, dadurch **gekennzeichnet,** daß die Zwischenlagerung bei einer Temperatur im Bereich von -15 bis -50°C durchgeführt wird.

13. Verfahren nach einem der Ansprüche 8 bis 12, dadurch **gekennzeichnet,** daß die Gefriertrocknung bei einem Druck im Bereich von 0,1 bis 50 mbar und bei einer solchen Temperatur durchgeführt wird, daß eine Produkttemperatur von +45°C nicht überschritten wird.

14. Trockenprodukt nach einem der Ansprüche 1 bis 7 zur Verwendung als Arzneimittel, Diätetikum oder als Zusatz zu Nahrungsmitteln.

15. Verwendung des Produktes nach einem der Ansprüche 1 bis 7 als Futter oder Futterzusatzstoff.

16. Verwendung des Produktes nach einem der Ansprüche 1 bis 7 als Halbfertigerzeugnis.

17. Verwendung des Produktes nach einem der Ansprüche 1 bis 7 als im Mund quellfähige Schlankheitskost.

## Claims

1. A dry solid product storable at room temperature and reconstitutable by saliva with a lactulose content of less than 0.5%, based on the dry product, for direct consumption on the basis of milk or milk products, carbohydrates, optionally other additives and with a content of lactic acid bacteria, characterized in that the content of lactic acid bacteria is at least 10⁸/g substance and in that the product has a finely porous outer structure so that the lactic acid bacteria remain viable over relatively long storage times.

2. A dry product as claimed in claim 1, characterized in that it contains milk of any fat content, milk concentrates, such as concentrated skimmed milk or whole milk, products enriched with soya protein, such as soya milk and milk-like nutrient media, or mixtures of these compounds as milk or the milk product.

3. A dry product as claimed in claim 1 or 2, characterized in that it contains sugar, starch, starch products, including starch degradation products, cereals in size-reduced form, vegetables or mixtures of these compounds as carbohydrates.

4. A dry product as claimed in claim 3, characterized in that it contains glucose, sucrose, fructose, lactose, sugar substitutes or mixtures of these compounds as sugars.

5. A dry product as claimed in at least one of claims 1 to 4, characterized in that the content of lactic acid bacteria is in the range from 10⁸/g to 10¹²/g.

6. A dry product as claimed in at least one of claims 1 to 5, characterized in that the content of lactic acid bacteria after storage for at least 3 months at room temperatures is in the range from 10⁸/g to 10¹⁰/g.

7. A dry product as claimed in at least one of claims 1 to 6, characterized in that it is in the form of slices, chips, sticks, figures, optionally provided with a coating.

8. A process for the production of the product claimed in any of claims 1 to 7 comprising preparing an aqueous basic mixture based on milk or milk products, carbohydrates and optionally other additives, homogenization, heating, cooling, inoculation, incubation and flash freezing with liquid nitrogen, characterized in that the mixture - incubated in an inert gas atmosphere - is flow-formed, the formed product is flash- or shock-frozen in an inert gas atmosphere, the frozen product is collected in an inert gas atmosphere, optionally stored temporarily in an inert gas atmosphere, freeze-dried in an inert gas atmosphere and packed in gas- and aroma-tight packs in an inert gas atmosphere.

9. A process as claimed in claim 8, characterized in that nitrogen is used as the inert gas in all stages.

10. A process as claimed in claim 8 or 9, characterized in that the flash freezing step is carried out so that the product has a core temperature of at least -15°C.

11. A process as claimed in any of claims 8 to 10, characterized in that the flash freezing step is carried out so that the product has a core temperature of -15 to -50°C.

12. A process as claimed in any of claims 8 to 11, characterized in that the temporary storage is carried out at a temperature of -15 to -50°C.

13. A process as claimed in any of claims 8 to 12, characterized in that the freeze drying step is carried out under a pressure of 0.1 to 50 mbar and at such a temperature that a product temperature of +45°C is not exceeded.

14. A dry product as claimed in any of claims 1 to 7 for use as a medicament, dietetic or food additive.

15. The use of the product claimed in any of claims 1 to 7 as an animal food or animal food additive.

16. The use of the product claimed in any of claims 1 to 7 as a semi-finished product.

17. The use of the product claimed in any of claims 1 to 7 as a slimming food swellable in the mouth.

## Revendications

1. Produit sec de forme solide, pouvant être reconstitué par la salive et stocké à la température ambiante, ayant une teneur en lactulose de moins de 0,5 % en poids, par rapport au produit sec, destiné à être consommé directement, à base de lait ou de produits lactés, de glucides, éventuellement d'autres additifs, et contenant des bactéries de l'acide lactique, caractérisé en ce que la teneur en bactéries de l'acide lactique est d'au moins 10⁸/g de substance et que le produit possède une structure externe à pores fins, grâce à quoi les bactéries de l'acide lactique restent viables pendant une durée de stockage assez longue.

2. Produit sec selon la revendication 1, caractérisé en ce qu'il contient, comme lait ou produit lacté, du lait à tous les taux de matières grasses, du lait concentré, comme du lait écrémé concentré ou du lait entier concentré, des produits enrichis en protéines du soja, comme du lait au soja, et des milieux nutritifs analogues au lait, ou des mélanges de ces composés.

3. Produit sec selon la revendication 1 ou 2, caractérisé en ce qu'il contient, comme glucides, du sucre, de l'amidon, des produits de l'amidon, y compris des produits de décomposition de l'amidon, des céréales sous la forme de petits morceaux, des végétaux, ou des mélanges de ces composés.

4. Produit sec selon la revendication 3, caractérisé en ce qu'il contient, comme sucre, du glucose, du saccharose, du fructose, du lactose, des substituts du sucre, ou des mélanges de ces composés.

5. Produit sec selon au moins une des revendications 1 à 4, caractérisé en ce que la teneur en bactéries de l'acide lactique est comprise entre 10⁸/g et 10¹²/g.

6. Produit sec selon au moins une des revendications 1 à 5, caractérisé en ce que la teneur en bactéries de l'acide lactique après un stockage d'au moins 3 mois à la température ambiante est comprise entre 10⁸/g et 10¹⁰/g.

7. Produit sec selon au moins une des revendications 1 à 6, caractérisé en ce qu'il se présente sous la forme de rondelles, de pastilles, de bâtonnets, de figures, éventuellement pourvu d'un enrobage.

8. Procédé de préparation du produit selon une des revendications 1 à 7, comprenant la préparation d'un mélange aqueux de base, à base de lait ou de produits lactés, de glucides et éventuellement d'autres additifs, son homogénéisation, son chauffage, son refroidissement, son inoculation, son incubation et sa congélation éclair avec de l'azote liquide, caractérisé en ce qu'on fait incuber le mélange dans une atmosphère de gaz inerte et on le façonne par fluage, on congèle le produit façonné par congélation éclair ou ultra-rapide dans une atmosphère de gaz inerte, on rassemble le produit congelé dans une atmosphère de gaz inerte, éventuellement on le stocke temporairement dans une atmosphère de gaz inerte, on le lyophilise dans une atmosphère de gaz inerte et on l'emballe de manière étanche aux gaz et aux arômes, dans une atmosphère de gaz inerte.

9. Procédé selon la revendication 8, caractérisé en ce qu'on utilise de l'azote comme gaz inerte dans toutes les étapes.

10. Procédé selon la revendication 8 ou 9, caractérisé en ce qu'on effectue la congélation éclair de manière que le coeur du produit atteigne une température d'au moins -15°C.

11. Procédé selon une des revendications 8 à 10, caractérisé en ce qu'on effectue la congélation éclair de manière que le coeur du produit atteigne une température comprise entre -15 et -50°C.

12. Procédé selon une des revendications 8 à 11, caractérisé en ce qu'on effectue le stockage intermédiaire à une température comprise entre -15 et -50°C.

13. Procédé selon une des revendications 8 à 12, caractérisé en ce qu'on effectue la lyophilisation à une pression de 0,1 à 50 mbar et à une température telle que la température du produit ne dépasse pas +45°C.

14. Produit sec selon une des revendications 1 à 7, pour une utilisation comme médicament, produit diététique ou comme produit d'appoint à des aliments.

15. Utilisation du produit selon une des revendications 1 à 7, comme fourrage ou produit d'appoint à du fourrage.

16. Utilisation du produit selon une des revendications 1 à 7, comme produit semi-fini.

17. Utilisation du produit selon une des revendications 1 à 7, comme aliment amaigrissant pouvant gonfler dans la bouche.
